# EUROPEAN PATENT SPECIFICATION

(11) **EP 3 119 416 B1**
(45) Date of publication and mention of the grant of the patent: **20.12.2017**
(21) Application number: 16722424.5
(22) Date of filing: 24.03.2016
(51) Int. Cl.: A61K 38/08, A61P 25/22, A61P 25/24

(54) **THERAPEUTIC USE OF ADIPOKINETIC HORMONE/RED PIGMENT-CONCENTRATING HORMONE (AKH/RPCH) FAMILY OF PEPTIDES**
THERAPEUTISCHE VERWENDUNG EINER PEPTIDFAMILIE AUS ADIPOKINETISCHEM HORMON/ ROTEM PIGMENT-KONZENTRIERENDEM HORMON (AKH/RPCH)
UTILISATION THÉRAPEUTIQUE DE LA FAMILLE D'HORMONES PEPTIDIQUES ADIPOCINÉTIQUES/CONCENTRANT LE PIGMENT PHYCOÉRYTHRINE (AKH/RPCH)

(30) Priority: 26.03.2015 TR 201503694
(43) Date of publication of application: 25.01.2017
(73) Proprietor: Mutlu, Oguz, Besiktas/Istanbul (TR)
(72) Inventor: Mutlu, Oguz, Besiktas/Istanbul (TR)
(74) Representative: Mutlu, Aydin
(86) International application number: PCT/TR2016/050077
(87) International publication number: WO 2016/153453

(56) References cited:
- OGUZ MUTLU ET AL: "Effects of chronic administration of adipokinetic and hypertrehalosemic hormone on animal behavior, BDNF, and CREB expression in the hippocampus and neurogenesis in mice", FUNDAMENTAL & CLINICAL PHARMACOLOGY., vol. 30, no. 1, 23 February 2016 (2016-02-23), pages 4-13, XP055294814, FR ISSN: 0767-3981, DOI: 10.1111/fcp.12165
- KAUFMANN C ET AL: "The adipokinetic hormone system in Culicinae (Diptera: Culicidae): Molecular identification and characterization of two adipokinetic hormone (AKH) precursors from Aedes aegypti and Culex pipiens and two putative AKH receptor variants from A. aegypti", INSECT BIOCHEMISTRY AND MOLECULAR BIOLOGY, ELSEVIER SCIENCE LTD, GB, vol. 39, no. 11, 1 November 2009 (2009-11-01), pages 770-781, XP026777041, ISSN: 0965-1748, DOI: 10.1016/J.IBMB.2009.09.002 [retrieved on 2009-09-11]
- GADE G ET AL: "A novel peptide in the AKH/RPCH family isolated from the corpora cardiaca of the Emperor dragonfly, Anax imperator", PEPTIDES, ELSEVIER, AMSTERDAM, NL, vol. 15, no. 1, 1 January 1994 (1994-01-01), pages 1-6, XP023982076, ISSN: 0196-9781, DOI: 10.1016/0196-9781(94)90162-7 [retrieved on 1994-01-01] cited in the application
- DALIBOR KODRIK ET AL: "A new member of the AKH/RPCH family that stimulates locomotory activity in the firebug, Pyrrhocoris apterus (Heteroptera)", INSECT BIOCHEMISTRY AND MOLECULAR BIOLOGY., vol. 30, no. 6, 1 June 2000 (2000-06-01), pages 489-498, XP055294799, GB ISSN: 0965-1748, DOI: 10.1016/S0965-1748(00)00025-4
- OGUZ MUTLU ET AL: "Effects of homeopathic Anax imperator on behavioural and pain models in mice", HOMEOPATHY, vol. 104, no. 1, 1 January 2015 (2015-01-01), pages 15-23, XP055294663, AMSTERDAM, NL ISSN: 1475-4916, DOI: 10.1016/j.homp.2014.05.002

## Description

### Technical Field of the Invention

The present invention is related to a use of Adipokinetic hormone/Red pigment-concentrating hormone (AKH/RPCH) family of peptides. The current invention particularly relates to Adipokinetic hormone/Red pigment-concentrating hormone (AKH/RPCH) family of peptides for use in the treatment of disease selected from the group consisting of depression, anxiety, stress disorders and locomotor disorders in human and animals. Among the stress disorders being one of the psychiatric diseases, posttraumatic stress disorders are particularly preferred.

### Background of the Invention

It is possible to find detailed information about the presence of peptides in insects especially about insect neuropeptides in literature. Concerning the peptides present in the insects, a categorization such as the following can be made: peptides involved in homeostasis and metabolism, peptides regulating reproduction, growth and development and peptides modifying spontaneous muscle contractions. A great variety of processes including metabolic, behavioural, developmental or reproductive in character in insects is known to be influenced or regulated by neuropeptides.

One of the major neuropeptide groups is the adipokinetic and hypertrehalosaemic peptides which belong to the AKH/RPCH family of peptides. Adipokinetic or hyperlipeamic effect is the increase of haemolymph lipid (diacylglycerol) concentration. Hypertrehalosaemic effect is the increase of haemolymph carbohydrates (sugar trehalose). Adipokinetic and hypertrehalosaemic peptides control fat, carbohydrate and protein metabolism. The structural similarity of adipokinetic and hypertrehalosaemic peptides to red pigment-concentrating hormone from the shrimp Pandalus borealis (Rab-RPCH) is the reason of naming this group of peptides as the AKH/RPCH family of peptides.

About 30 different peptides are known at present which renders this family as one of the largest family of peptides. All members are from 8 to 10 amino acids long, N-terminally blocked by a pyroglutamate residue and C- terminally blocked by an amide. At position 4 (Phe or Tyr) and 8 (Trp) aromatic residues are present; most variations in constituent amino acids are conservative.

Besides the hyperlipaemic and hypertrehalosaemic effects, the following are indicated as the major activities of peptides of the AKH/RPCH family.
- Stimulation of the frequency of the heart beat in Periplaneta Americana
- Increase in muscle tone and frequency of contraction of the spontaneous activity of the isolated leg of a locust
- Inhibition of protein synthesis in L. migratoria and in cricket Acheta domesticus
- Inhibition of fatty acid synthesis in S. gregaria
- Inhibition of RNA synthesis in fat body of L. migratoria

In conclusion, the AKH/RPCH peptides exert multiple physiological effects including mainly acting on the metabolic status of the body in various insect model systems. Accordingly, many physiological research is performed on the functions of adipokinetic hormones in locust during flight. Said hormones have direct effects on the mobilization of carbohydrates and lipids and/or the utilization of such substrates by flight muscles, but have additional indirect effects on the transport of lipids as lipoproteins to flight muscles and on the enzyme system of lipoprotein lipase in the flight muscles. This enzyme is responsible for unloading of the diacylglycerol from lipoproteins and making it finally available for oxidation to power the contraction of the flight muscle (Gade G., The Explosion of Structural Information on Insect Neuropeptides, in "Progress in the Chemistry of Organic Natural Products no. 71", 1997).

Adipokinetic hormones and hypertrehalosaemic hormones of insects comprise a family of peptide hormones that primarily regulate the levels of energy metabolites, such as trehalose (disaccharide), diacylglycerol and proline that circulate in the haemolymph. These peptide hormones are products of neurosecretory neurons located in the corpora cardiaca, neuroendocrine glands attached to the brain. The structural organization of the insect corpora cardiaca is similar to the hypothalamus-neurohypophysis of the vertebrate endocrine system (Gade, G., Heather G., Adipokinetic and hypertrehalosaemic neurohormones in "Marco Biomedical and Life Sciences Encyclopaedia of Entomology", Springer reference).

Neurosecretory cells in the corpus cardiacum of insects synthesize a set of hormones that are called adipokinetic, hypertrehalosaemic or hyperprolinaemic depending on the insect in question. These neuropeptides act as hormones, thus are neurohormones, and are especially needed when the oxidative metabolism of insects is very high; for example, when flight muscles contract maximally and over longer periods. Hence, insects need large amounts of energy which eventually has to be mobilized from stores in the fat body (Gade, G., The importance of small peptide hormones for regulation of energy metabolism in insects, Seminar, 2011, Cape Town).

Adipokinetic hormones (AKHs) are metabolic neuropeptides, mediating mobilization of energy substrates from the fat body in many insects. Studies using transgenic manipulations of the dAkh gene have demonstrated that AKH induces both hypertrehalosaemia and hyperlipaemia. Moreover, it is known that AKH peptides have excitatory effects on motor neurons. Evidence from studies in other insects supports the central role of AKH for locomotion.

Energy homeostasis is a fundamental property of animal life, providing a genetically fixed balance between fat storage and mobilization. The importance of body fat regulation is emphasized by dysfunctions resulting in obesity and lipodystrophy in humans. Combined genetic, physiological, and biochemical analyses provide in vivo evidence that AKH receptor (AKHR) is as important for "chronic accumulation and acute mobilization of storage fat" as is in the "Brummer lipase", the homolog of mammalian adipose triglyceride lipase (ATGL). Simultaneous loss of Brummer and AKHR causes extreme obesity and blocks acute storage-fat mobilization in flies (Grönke, S., Müller, G., Hirsch, J., Fellert, S., Andreou, A., Haase, T., Jäckle, H. and Kühnlein, R. P. (2007). Dual lipolytic control of body fat storage and mobilization in Drosophila. PLoS Biol. 5(6): e137).

The insect adipokinetic hormones (AKHs) are a large family of peptide hormones that are involved in the mobilization of sugar and lipids from the insect fat body during energy-requiring activities such as flight and locomotion. They also contribute to haemolymph sugar homeostasis. Interestingly, the insect AKH receptors are structurally and evolutionarily related to the gonadotropin-releasing hormone receptors from vertebrates (Staubli, F., et al. (2002). Molecular identification of the insect adipokinetic hormone receptors. Proc. Natl. Acad. Sci. 99(6): 3446-51).

Using a heterologous (in locusts and cockroaches) and a homologous bioassay, the neuropeptide pGlu-Val-Asn-Phe-Ser-Pro-Ser-Trp-NH₂ was isolated from extracts of corpora cardiaca of the Emperor dragonfly, *Anax imperator.* Low concentrations of the synthetic peptide injected into the Emperor dragonfly increased the haemolymph lipid concentration, suggesting a possible role of the peptide in lipid homeostasis during flight. Therefore, it is named Ani-AKH, *Anax imperator* adipokinetic hormone (Gade G, Janssens MP, Kellner R. A novel peptide in the AKH/RPCH family isolated from the corpora cardiaca of the Emperor dragonfly, Anax imperator. Peptides, Vol. 15, No. 1, pp. 1-6, 1994).

Adipokinetic hormones (AKHs) mobilize lipids, carbohydrates and/or proline from insect fat body stores. In addition, AKHs inhibit lipid and protein synthesis in the fat body. In a previous study, the effects of the injected AKH on the formation of energy reserves in the fat body and on egg production were measured. In comparison to water-injected control animals, lipid and protein content in the fat body of the AKH-injected crickets was significantly reduced. These results suggest an inhibitory effect of AKH on the formation of lipid reserves and protein stores. The content of glycogen and free carbohydrate in the fat body was significantly higher in the AKH-injected animals. The most pronounced effect of the AKH-injections was a significant reduction of ovary mass, due to the retarded maturation of the oocytes and the significantly lower number of terminal oocytes produced. Consequently, it is concluded that AKH inhibits egg production indirectly by interference with the formation of energy stores in the fat body that are mobilized to fuel egg production (Lorenz MW. Adipokinetic hormone inhibits the formation of energy stores and egg production in the cricket Gryllus bimaculatus. Comp. Biochem. Physiol. B. Biochem. Mol. Biol. 2003 Oct; 136(2):197-206).

In a recent study adipokinetic neuropeptides from the corpora cardiaca of the major families of all three suborders of the Odonata were identified. Sequence assignment revealed that the investigated Odonata species always contain only one adipokinetic peptide. This is always an octapeptide (Gade G, Marco HGJ. The adipokinetic hormones of Odonata: a phylogenetic approach. Insect Physiol. 2005 Mar; 51(3):333-41).

In a previous study, it is suggested that adipokinetic hormone may contribute to the neuronal function in the human central nervous system. This study reveals that an antiserum raised against locust adipokinetic hormone I can display a considerable quantity of adipokinetic hormone-like immunoreactivity in the human cerebrospinal fluid. As a result of the gel permeation and high performance liquid chromatography, the main immunoreactive component in the cerebrospinal fluid coeluted with adipokinetic hormone I. These results suggest that adipokinetic hormone may contribute to the neuronal function in the human central nervous system (Suzuki H, Sato S, Tsuchiya T, Suzuki Y, Muramatsu H, Ishihara N, Shimoda S. Identification and characterization of adipokinetic hormone (Locusta migratoria)-like immunoreactivity in the human cerebrospinal fluid, Biochemical and biophysical research communications; 1989; 163 (1): 534-540).

In another study, it has been identified the first insect AKH receptors, namely those from the fruit fly *Drosophila melanogaster* and the silkworm *Bombyx mori.* The results of this study represent a breakthrough for insect molecular endocrinology, because it will lead to the cloning of all AKH receptors from all model insects used in AKH research, and, therefore, to a better understanding of AKH heterogeneity and actions. It is expressed in this study that the insect AKH receptors are structurally and evolutionarily related to the gonadotropin-releasing hormone receptors from vertebrates (Staubli et al., Molecular identification of the insect adipokinetic hormone receptors, Proc. Natl. Acad. Sci. USA. 2002 Mar 19;99(6):3446-51.).

In a different study, the effect of pharmaceutically active ingredients on insects were investigated and the effects of five biogenic amines including norepinephrine, dopamine, octopamine, serotonin and histamine on the locomotory activity and mobilization of lipids in the adult females of the firebug, Pyrrhocoris apterus (L.) were researched. The results demonstrated that all five tested biogenic amines mobilized the fat body lipids, but only norepinephrine and dopamine were capable to enhance the walking activity simultaneously with an elevation of the lipid level in the haemolymph. Those two amines had no effect on the level of AKHs in CNS, but modulated the AKHs level in haemolymph: norepinephrine increased it, while dopamine decreased it. The results indicate an apparent feedback between AKH characteristics and dopamine and norepinephrine actions occurring in this insect species. While the stimulatory effects of norepinephrine on lipid mobilization and walking activity could involve the release of bug's own AKHs, dopamine probably employs an independent stimulatory pathway. (Socha R, Kodrík D, Zemek R. Stimulatory effects of bioamines norepinephrine and dopamine on locomotion of Pyrrhocoris apterus (L.): is the adipokinetic hormone involved? Comp. Biochem. Physiol. B. Biochem. Mol. Biol. 2008 151(3):305-10)).

In addition to the studies performed on insects, there are also studies performed on mice or rat. One of these is the examination of central action of periplanetin CC-1 (Pea-HrTH) (Glp-Val-Asn-Phe-Ser-Pro-Asn-TrpNH2), octapeptide of the insect adipokinetic hormone family (AKH-family), isolated from American cockroach-Periplaneta americana in Albino Swiss mice (20-25 g). Upon injection of this peptide to the mice, it is understood in the "writhing syndrome" and "hot plate" tests that the peptide showed strong analgesic activity. In addition, periplanetin CC-1 decreased the threshold for tonic seizures and increased mortality in pentetrazole-induced seizures, having no influence on the electric convulsions. CC-1 is known to be a glucagon-like AKH in insects. However, administered intracerebroventricularly to mice, it did not increase the peripheral blood glucose level. The obtained results indicate that periplanetin CC-1 shows a biological action in vertebrates, but its metabolic effects are different than in insects (Sieklucka-Dziuba M, Morawska D, Lombarska-Sliwińska D, Konopińska D, Kleinrok Z. Central action of insect neuropeptide, periplanetin CC-1, in mice. Pol J Pharmacol. 1997; 49(1):43-8).

An antiserum was raised to adipokinetic hormone (AKH), a 10-amino-acid-residue peptide found in the arthropod Locusta migratoria. The antiserum demonstrated not only immunocytochemical reaction with some other arthropod species, but also stained many areas of the rat CNS, certain islet cells of the pancreas, and some anterior pituitary cells. The pattern of staining was unlike that for any known rat neuropeptide or hormone. With the antiserum used as the detection system, HPLC and high-voltage electrophoresis yielded two peptides that were purified to homogeneity from rat hypothalamic median eminence. These peptides have unique amino acid compositions, indicating they may be heretofore unknown rat neuropeptides (Schueler PA, Elde RP, Herman WS, Mahoney WC. Identification and initial characterization of adipokinetic hormone-like immunoreactive peptides of rat origin. J Neurochem. 1986 Jul; 47(1):133-8).

In a study performed in rat, a novel peptidergic system was identified in the rat central nervous system by using an antiserum to locust adipokinetic hormone I. Immunoreactive fibers were present in the hypothalamic median eminence and periventricular nucleus and the spinal cord dorsal horn, intermediolateral cell column and sacral parasympathetic nucleus. Immunoreactive cells were present in the dorsal gray commissure of lumbosacral spinal cord, the hypothalamic periventricular nucleus and cerebral cortex. (Sasek CA, Schueler PA, Herman WS, Elde RP. An antiserum to locust adipokinetic hormone reveals a novel peptidergic system in the rat central nervous system. Brain Res. 1985 Sep 16; 343(1):172-5).

There are many studies in the prior art directed more particularly to the AKH family. In one of these studies, it is determined that AKHs respond to stress situations including oxidative stress and insecticide treatment to the insects elicited an intensive AKH increase in haemolymph. It is also observed in the same study that when AKH is applied to insects by mixing with insecticides, it is observed that the carbon dioxide production and the metabolic rate are increased thus bug mortality is also increased. It is thought that this enhanced effect of the insecticides probably resulted from the stimulatory role of AKH on bug metabolism (Velki M., Kodrík D., Večeřa J., Hackenberger B.K., Socha R. Oxidative stress elicited by insecticides: a role for the adipokinetic hormone. Gen. Comp. Endocrinol. 2011; 172(1): 77-84).

In another study, excitability and locomotor activity of male and female last instar larvae and adults of the two-spotted cricket were measured. It was concluded that AKH could be responsible for linking the endogenous clock output with the cyclic changes in locomotor activity. Furthermore, AKH might serve to synchronise metabolism and behaviour to optimise larval development and reproduction (Fassold K, El-Damanhouri HI, Lorenz MW. Age-dependent cyclic locomotor activity in the cricket, Gryllus bimaculatus, and the effect of adipokinetic hormone on locomotion and excitability. J. Comp. Physiol. A Neuroethol. Sens. Neural. Behav. Physiol. 2010; 196(4): 271-83).

The functions of adipokinetic hormones in lipid metabolism are also subject to many patent applications as well. For instance, the US patent application US 6,852,693 B2 is related to a method for using polypeptide compounds based on the structures of insect peptides of the adipokinetic hormone family to mobilize lipids in humans. Said application discloses these compositions and peptides could be useful for modulating human body weight, such as inducing weight loss and methods for identifying other compounds effective for modulating lipid mobilization in humans.

As it can be seen, many features of the AKHs, a large family of peptides, are revealed by the studies performed so far and today investigations are still on going. The present invention is related to a use of adipokinetic hormone/red pigment-concentrating hormone (AKH/RPCH) family of peptides which could be surprisingly effective in the treatment of diseases including some psychiatric diseases and/or locomotor disorders.

### Disclosure of the Invention

The current disclosure relates to a use of a peptide of the adipokinetic hormone/red pigment-concentrating hormone (AKH/RPCH) family of peptides. It is particularly related to the use of said peptide in the treatment of a disease selected from psychiatric diseases including depression, anxiety, stress disorders and/or locomotor disorders or a combination thereof. Stress disorders of the psychiatric diseases are particularly posttraumatic stress disorders.

The present invention relates to the peptide hormones shown below:
**pGlu-Val-Asn-Phe-Xaa⁵-Pro-Ser-Trp-NH₂**
wherein Xaa⁵ residue can be selected among Ser and Thr residues, and
**pGlu-Leu-Thr-Phe-Ser-Pro-Asp-Trp-NH₂**
for use in the treatment of a disease selected from the group consisting of depression, anxiety, posttraumatic stress disorders and locomotion disorders:

### Brief Description of the Figures

**Figure 1a** illustrates the immobility time data obtained by administering intraperitoneally (i.p.) Ani-AKH (*Anax Imperator Mauricianus* AKH) at different doses for 14 days in the Forced Swim Test (FST) in mice. Data is indicated as ± standard error of the mean. (***p<0.001 compared to the control group)
**Figure 1b** illustrates the immobility time data obtained by administering intraperitoneally at different doses for 14 days Lia-AKH (*Libellula Auripennis* AKH) and Pht-HrTH (*Phormia Terrae-Novae* Hypertrehalosaemic hormone) in the Forced Swim Test (FST) in mice. Data is indicated as ± standard error of the mean. (***p<0.001 compared to the control group)
**Figure 2a** illustrates the % time spent in the open arms obtained upon administration of intraperitoneally at different doses Ani-AKH acutely before Elevated Plus-Maze Test (EPM). Data is indicated as ± standard error of the mean. (*p<0.05 compared to the control group).
**Figure 2b** illustrates the % open arm/total arm entries obtained upon administration of intraperitoneally at different doses Ani-AKH acutely in the Elevated Plus-Maze Test (EPM). Data is indicated as ± standard error of the mean. (*p<0.05 compared to the control group).
**Figure 2c** illustrates the % time spent in the open arms obtained upon administration of Ani-AKH chronically at different doses (i.p. for 14 days) in the Elevated Plus-Maze Test (EPM).
**Figure 2d** illustrates the % open arm/total arm entries obtained upon administration of Ani-AKH at different doses chronically (i.p. for 14 days) in the Elevated Plus-Maze Test (EPM).
**Figure 2e** illustrates the % time spent in the open arms obtained upon administration of Lia-AKH and Pht-HrTH at different doses chronically (i.p. for 14 days) in the Elevated Plus-Maze Test (EPM). Data is indicated as ± standard error of the mean. (*p<0.05 compared to the control group)
**Figure 2f** illustrates the % open arm/total arm entries obtained upon administration of Lia-AKH, Pht-HrTH at different doses chronically (i.p. for 14 days) before in the Elevated Plus-Maze Test (EPM). Data is indicated as ± standard error of the mean. (**p<0.01 and ***p<0.001 compared to the control group)
**Figure 3a** Effects of Ani-AKH given intraperitoneally at different doses for 14 days on total distance moved in the locomotor activity test. Data is indicated as ± standard error of the mean. (**p<0.01 compared to the control group)
**Figure 3b** Effects of Ani-AKH given intraperitoneally at different doses for 14 days on speed in the locomotor activity test. Data is indicated as ± standard error of the mean. (**p<0.01, compared to the control group)
**Figure 3c** Effects of Lia-AKH. Pht-HrTH given intraperitoneally at different doses for 14 days on total distance moved in the locomotor activity test. Data is indicated as ± standard error of the mean. (*p<0.05, compared to the control group)
**Figure 3d** Effects of Lia-AKH, Pht-HrTH given intraperitoneally at different doses for 14 days on speed in the locomotor activity test. Data is indicated as ± standard error of the mean. (*p<0.05, compared to the control group)

### Detailed Description of the Invention

The present disclosure is related to the use of adipokinetic hormone/red pigment-concentrating hormone (AKH/RPCH) family of peptides in the treatment of diseases selected from psychiatric diseases including depression, anxiety, stress disorders and/or locomotor disorders or a combination thereof. Among the stress disorders being one of the psychiatric diseases, posttraumatic stress disorders are particularly preferred.

The term "peptide" refers to an amino acid (aa) polymer of adipokinetic hormone/red pigment-concentrating hormone (AKH/RPCH) family. Said peptide signifies preferably a peptide from 8 to 10 amino acids long, N-terminally blocked by a pyroglutamate residue and C- terminally blocked by an amide. More particularly, said peptide refers to a peptide having aromatic residues at position 4 (Phe or Tyr) and 8 (Trp). Particularly, the present invention is about a member of Adipokinetic hormone/Red pigment-concentrating hormone (AKH/RPCH) family of peptide hormones.

"A pharmaceutical composition for use in the treatment of psychiatric diseases including depression, anxiety, posttraumatic stress disorders and/or locomotor disorders or a combination thereof" is related to any composition comprising the peptide defined above which prevents, ameliorates or cures said diseases.

The peptide hormones of the present invention are as follows:
**pGlu-Val-Asn-Phe-Xaa⁵-Pro-Ser-Trp-NH₂**
wherein Xaa⁵ residue is selected among Ser and Thr amino acids, and
**pGlu-Leu-Thr-Phe-Ser-Pro-Asp-Trp-NH₂**
for use in the treatment of a disease selected from the group consisting of depression, anxiety, posttraumatic stress disorders and locomotion disorders.

In another aspect, the present invention relates to a pharmaceutical composition as defined in claim 2 and at least one pharmaceutically acceptable excipient. Said pharmaceutical composition is suitable for use in human and animals.

Our study using a mice model related to the adipokinetic hormone/red pigment-concentrating hormone peptide family is presented below. The peptides used in this study is presented in Table 1 with their code names and amino acid sequences. The peptides synthesized chemically are obtained from the companies TRC (Canada) and SciLight Biotechnology (China).

**Table 1 - Adipokinetic hormone/red pigment-concentrating hormone peptides used in mice models**

| **AKH/RPCH Family - Code Name** | **CAS No** | **Amino acid Sequence - Chemical Name** |
|---|---|---|
| Ani-AKH (Anax Imperator Mauricianus) | 154512-22-8 | pGlu-VNFSPSW-NH2 pGlu-Val-Asn-Phe-Ser-Pro-Ser-Trp-NH2 *5-oxo-L-prolyl-L-valyl-L-asparaginyl-L-phenylalanyl-L-seryl-L-prolyl-L-seryl-L-Tryptophanamide* |
| Lia-AKH (Libellula Auripennis) | 129536-34-1 | PGlu-VNFTPSW-NH2 pGlu-Val-Asn-Phe-Thr-Pro-Ser-Trp-NH2 *5-oxo-L-prolyl-L-valyl-L-asparaginyl-L-phenylalanyl-L-threonyl-L-prolyl-L-seryl-L-Tryptophanamide* |
| Pht-HrTH Hypertrehalosaemic hormone (Phormia Terrae-Novae) | 129204-82-6 | pGlu-LTFSPDW-NH2 pGlu-Leu-Thr-Phe-Ser-Pro-Asp-Trp-NH2 *5-oxo-L-prolyl-L-leucyl-L-threonyl-L-phenylalanyl-L-seryl-L-prolyl-L-alfa-aspartyl-L-Tryptophanamide* |

### Examples

### The study of Adipokinetic hormone/red pigment-concentrating hormone peptides in mice models

### 1. Animals

Male, inbred BALB/c ByJ mice (Uludag University, Bursa, Turkey), 7-8 weeks old at their arrival to the laboratory, were used in this study. They were kept in the laboratory for two weeks before the onset of the experiments. Mice were maintained under standard laboratory conditions (12-h light: 12-h dark cycle, lights on 07:00 h, T=21±1°C). All animals received food and water ad libitum. All procedures described in this assay were conducted in accordance with the European Community Council's Directive for the ethical treatment of animals (86/609/EEC) and with the approval of the Kocaeli University Medical Faculty (7/3/2014).

### 2. Experimental groups and drug administration

In the FST, EPM, and locomotor activity tests, the animals were treated with Ani-AKH (1 and 2 mg/kg) (n=9-10), Lia-AKH (1 and 2 mg/kg) (n=10-12) and Pht-HrTH (1 and 2 mg/kg) (n=10-12) or vehicle (saline with 1% DMSO) (n= 10) for 14 days (chronically) and the drugs were administered intraperitoneally (i.p.) 60 min. before each test in a volume of 0.1 ml/10 g body weight. Separate group of animals used in each test.

In addition, in the EPM (acute) test, Ani- AKH (0.5 and 1 mg/kg) (n= 10 per group) was also administered, just for one time, before the test to investigate the acute effect of the hormone.

### 3. Forced Swim Test (FST)

The FST employed was similar to a previously described method (Porsolt et al, 1977). Briefly, the mice were dropped individually into Plexiglas cylinders (height 25 cm, diameter 10 cm) containing 10 cm of water maintained at 23-25°C and left there for 6 min. Because this is a situation from which they cannot escape, the animals rapidly become immobile, that is, floating in an upright position and making only small movements to keep their heads above water. The duration of immobility was recorded during the last 4 min of the 6-min testing period.

### 4. Elevated Plus-Maze Test (EPM)

Anxiety related behaviour was measured by the elevated plus-maze test. Experiments were conducted in a dimly lit, semi-soundproof room, illuminated with table lamp (80 lux). Maze was made of wood and consisted of two open (29 cm long × 5 cm wide) and closed arms (29 cm × 5 cm with 15 cm high walls) forming a square cross with a 5 cm square center piece. In order to avoid falls the open arms was surrounded by a short (1 cm) Plexiglas edge. The maze was elevated 40 cm above the floor. Each mice was placed at the center of the maze, facing one of the open arms, and was allowed to explore the maze. The open-arm activity was evaluated as: 1) time spent in the open arms relative to the total time spent in the plus maze (300 s), expressed as a percentage 2) number of entries into the open arms relative to the total number of entries into both open and closed arms, expressed as a percentage. These values were accepted as indexes of anxiety in mice.

### 5. Locomotor Activity test

Locomotor activity was measured using an open field test in a square arena (40 x 40 x 40 cm box). The animal was placed in the center of the apparatus and behaviours were recorded for a period of 5 min using the Etovision-XT video tracking system. The locomotor activity was evaluated by measuring the total distance travelled (cm) in the apparatus and the speed (cm/s) of the animals.

### 6. Statistical Evaluation

The results of the FST, EPM, locomotor activity tests were evaluated by one-way ANOVA followed by Tukey's post-hoc test when significant differences were detected. The data are expressed as the mean values ± SEM. The differences were considered to be statistically significant when p was less than 0.05.

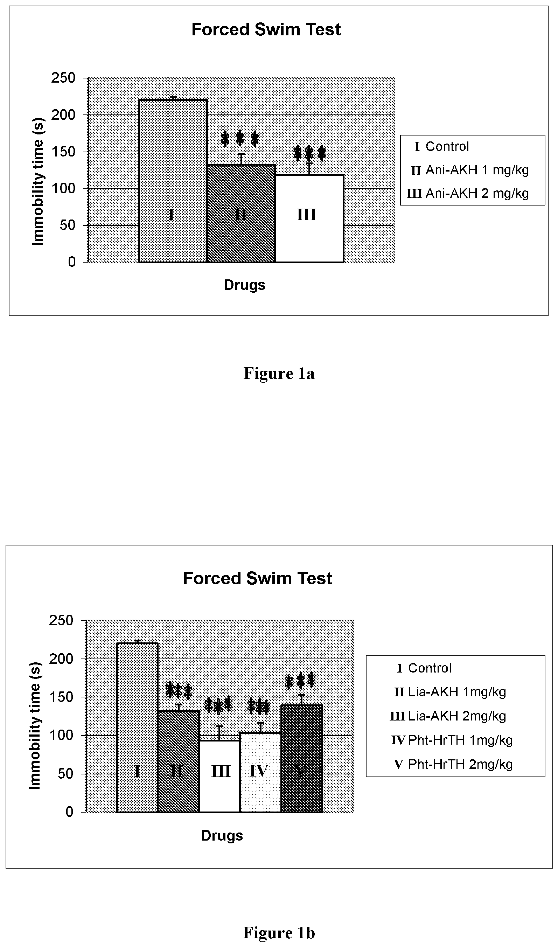

In the FST test, Ani-AKH (1 and 2 mg/kg) (p<0.001) significantly diminished immobility time compared to control group [F (2.26) = 20.04; p<0.0001; Fig 1a]. Lia-AKH (1 and 2 mg/kg) (p<0.001) and Pht-HrTH (1 and 2 mg/kg) (p<0.001) also significantly diminished immobility time compared to control group [F (4.43) = 16.27; p<0.0001; Fig 1b].

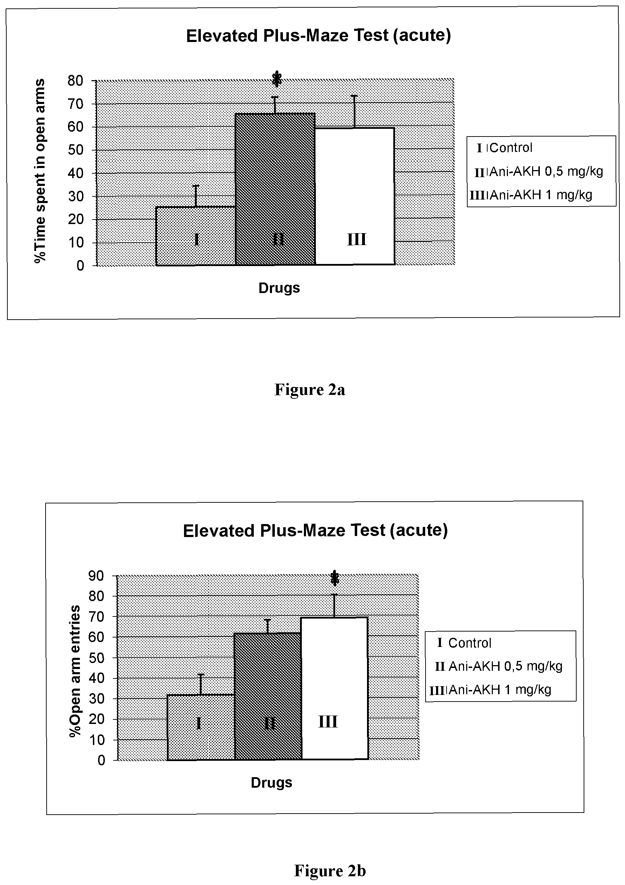

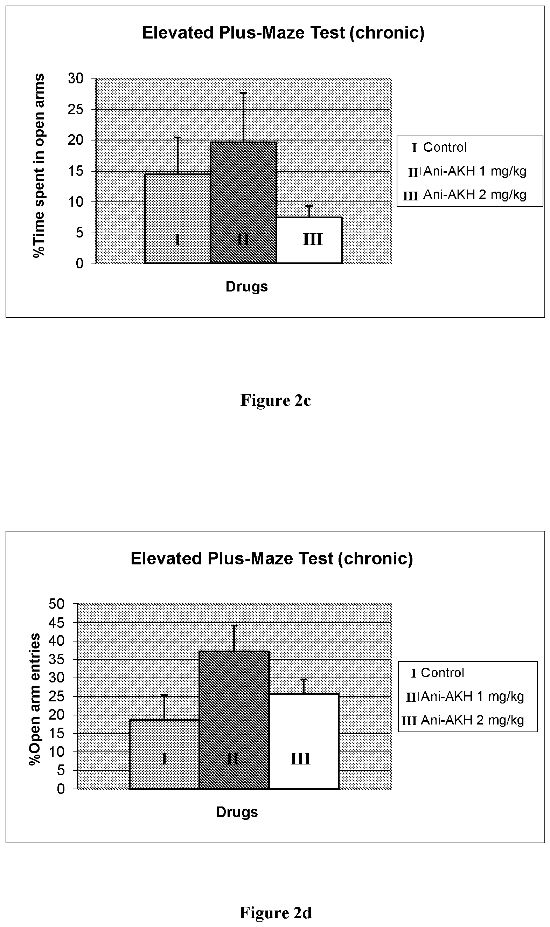

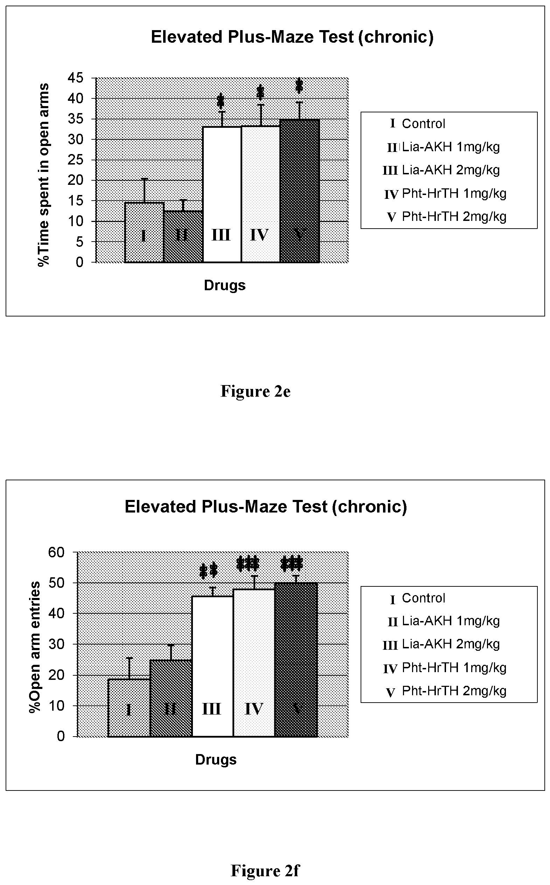

In the EPM test, Ani-AKH 0.5 mg/kg (p<0.05) when administered acutely significantly increased %time spent in the open arms [F (2.27) = 4.13; p=0.02; Fig 2a] and Ani-AKH 1 mg/kg (p<0.05) significantly increased %open arm/total arm entries [F (2.27) = 4.34; p=0.02; Fig 2b]. In the EPM test, Ani-AKH (1 and 2 mg/kg) didn't alter %time spent in the open arms [F (2.26) = 1.13; p = 0.33; Fig 2c] and %open arm/total arm entries [F (2.26) = 2.34; p=0.11; Fig 2d] when administered chronically. In the EPM test, Lia-AKH (2 mg/kg) (p<0.05) and Pht-HrTH (1 and 2 mg/kg) (p<0.05) when administered chronically significantly increased %time spent in the open arms [F(4,45)=6.10; p=0.0005; Fig 2e] while Lia-AKH (2 mg/kg) (p<0.01) and Pht-HrTH (1 and 2 mg/kg) (p<0.001) significantly increased %open arm/total arm entries [F(4,45)=10.23; p<0.0001; Fig 2f].

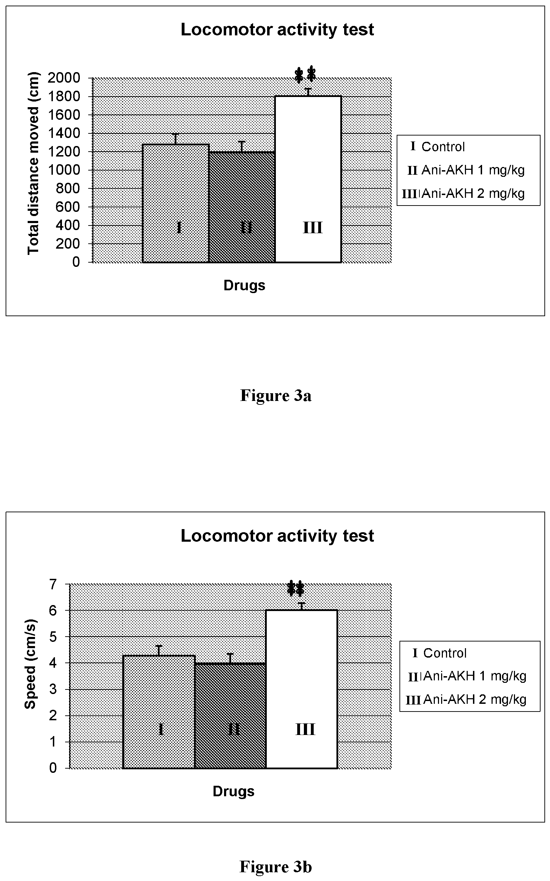

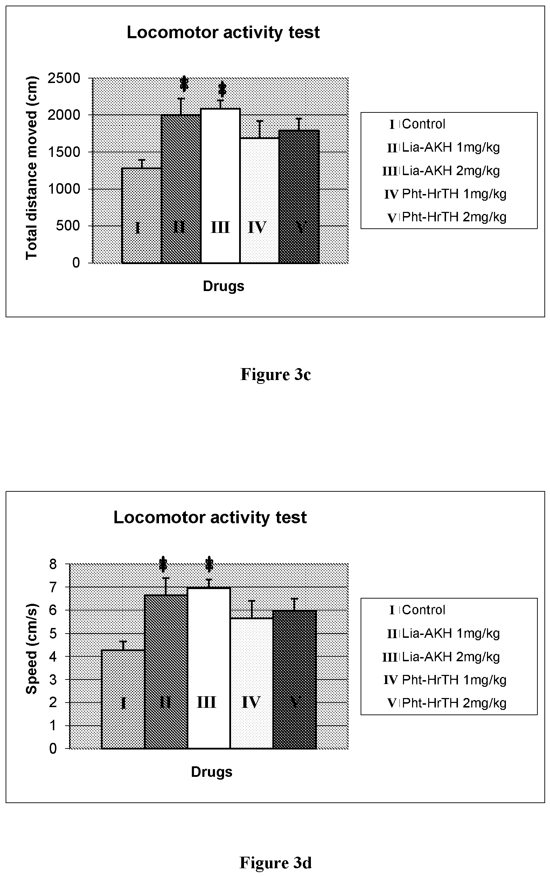

In the locomotor activity test, Ani-AKH (2 mg/kg) (p<0.01) increased the total distance moved compared to control [F (2.26) =9.94; p=0.0006; Fig 3a] and increased the speed of the animals compared to control [F (2.26) =9.81; p=0.0007; Fig 3b]. In the locomotor activity test, Lia-AKH (1 and 2 mg/kg) (p<0.05) and Pht-HrTH increased the total distance moved [F (4.49) =3.35; p=0.01; Fig 3c] and increased the speed of the animals [F (4.49) = 3.30; p=0.01; Fig 3d].

### 7. Results

Ani-AKH (0.5 and 1 mg/kg) when administered acutely had anxiolytic effects in the elevated plus maze test while Lia-AKH (2 mg/kg) and Pht-HrTH (1 and 2 mg/kg) had anxiolytic effects when given chronically in the elevated plus maze test.

Ani-AKH (1 and 2 mg/kg), Lia-AKH (1 and 2 mg/kg), Pht-HrTH (1 and 2 mg/kg) had antidepressant effects in the forced swimming test in male balb-c mice when injected intraperitoneally as 0.1ml/10g of body weight. According to these results, AKH/RPCH family peptides may be used in the treatment of a psychiatric illness like depression, anxiety and stress disorders such as posttraumatic stress disorder.

Ani-AKH (2 mg/kg) and Lia-AKH (1 and 2 mg/kg) and Pht-HrTH had locomotion enhancing effects in the locomotor activity test in male balb-c mice when injected intraperitoneally as 0.1ml/10g of body weight. According to these results, AKH/RPCH family peptides may be used in the treatment of the diseases related to locomotion system.

## Claims

1. A peptide hormone of the AKH/RPCH peptide family selected from the group of:
**pGlu-Val-Asn-Phe-Xaa⁵-Pro-Ser-Trp-NH₂**
wherein; Xaa⁵ residue is selected among Ser and Thr amino acids, and
**pGlu-Leu-Thr-Phe-Ser-Pro-Asp-Trp-NH₂**
for use in the treatment of a disease selected from the group consisting of depression, anxiety, posttraumatic stress disorders and locomotion disorders.

2. A pharmaceutical composition comprising the peptide hormone according to claim 1 for use in the treatment of a disease selected from the group consisting of depression, anxiety, posttraumatic stress disorders and locomotion disorders.

## Patentansprüche

1. Peptidhormon der AKH/RPCH-Peptidfamilie, das aus der Gruppe
**pGlu-Val-Asn-Phe-Xaa⁵-Pro-Ser-Trp-NH₂**
wobei der Rest Xaa⁵ aus den Aminosäuren Ser und Thr ausgewählt ist, und
**pGlu-Leu-Thr-Phe-Ser-Pro-Asp-Trp-NH₂**
ausgewählt ist, zur Verwendung bei der Behandlung einer Erkrankung, die aus der Gruppe ausgewählt ist, die aus Depressionen, Angstzuständen, posttraumatischen Belastungsstörungen und Fortbewegungsstörungen besteht.

2. Pharmazeutische Zusammensetzung, die das Peptidhormon gemäß Anspruch 1 umfasst, zur Verwendung bei der Behandlung einer Erkrankung, die aus der Gruppe ausgewählt ist, die aus Depressionen, Angstzuständen, posttraumatischen Belastungsstörungen und Fortbewegungsstörungen besteht.

## Revendications

1. Hormone peptide de la famille des peptides AKH/RPCH qui est sélectionnée dans le groupe de :
**pGlu-Val-Asn-Phe-Xaa⁵-Pro-Ser-Trp-NH₂**
dans lequel ; le résidu Xaa⁵ est sélectionné parmi les acides aminés Ser et Thr ; et
**pGlu-Leu-Thr-Phe-Ser-Pro-Asp-Trp-NH₂**
pour une utilisation dans le traitement d'une maladie sélectionnée dans le groupe consistant en la dépression, l'anxiété, les troubles liés au stress post-traumatique et les troubles de la locomotion.

2. Composition pharmaceutique comprenant l'hormone peptide selon la revendication 1, pour une utilisation dans le traitement d'une maladie sélectionnée dans le groupe consistant en la dépression, l'anxiété, les troubles liés au stress post-traumatique et les troubles de la locomotion.
